Europäisches Patentamt

**European Patent Office**

**Office européen des brevets**

(11) Numéro de publication: **0 264 329 B1**

# FASCICULE DE BREVET EUROPEEN

(45) Date de publication de fascicule du brevet:
**15.01.92**

(21) Numéro de dépôt: **87402246.0**

(22) Date de dépôt: **08.10.87**

(51) Int. Cl.⁵: **A61B 5/00**, A61B 5/04, A61B 5/103

(54) **Dispositif de surveillance de l'activite d'un organe du corps humain.**

(30) Priorité: **15.10.86 FR 8614328**

(43) Date de publication de la demande:
**20.04.88 Bulletin 88/16**

(45) Mention de la délivrance du brevet:
**15.01.92 Bulletin 92/03**

(84) Etats contractants désignés:
**AT BE CH DE GB IT LI NL SE**

(56) Documents cités:
**US-A- 3 978 847**
**US-A- 4 461 301**

**MEDICAL AND BIOLOGICAL ENGINEERING,
vol. 13, no. 2, mars 1975, pages 266-271,
Stevenage, GB; S. TONKOVIC et al.: "Une
application de l'analyse spectrale dans le
traitement des signaux electrosplanchnographiques"**

(73) Titulaire: **COMMISSARIAT A L'ENERGIE ATO-
MIOUE Etablissement de Caractère Scientifique Technique et Industriel
31/33, rue de la Fédération
F-75015 Paris(FR)**

Titulaire: **UNIVERSITE DE TOURS
3 rue des Tanneurs
F-37041 Tours Cédex(FR)**

(72) Inventeur: **Marquis, Ghislaine
Bât.BI, Les Millepertuis Avenue du Berry
F-91940 Les Ulis(FR)**
Inventeur: **Martin, André
22 Bd de Chinon Les Rosiers
F-37300 Joue-Les-Tours(FR)**
Inventeur: **Murat, Jean
27 rue Boisdenier
F-37000 Tours(FR)**
Inventeur: **Prulhière, Jean-Pierre
16 rue Fernand Marin
F-33000 Bordeaux(FR)**

IEEE TRANSACTIONS ON BIOMEDICAL ENGI-NEERING, vol. BME-32, no. 11, novembre 1985, pages 911-915, IEEE, New York, US; B.E. BELLAHSENE et al.: "An improved method for recording and analyzing the electrical activity of the human stomach"

IEEE TRANSACTIONS ON BIOMEDICAL ENGI-NEERING, vol. BME-29, no. 7, juillet 1982, pages 503-510, IEEE, New York, US; O. ROM-PELMAN et al.: "The measurement of heart rate variability spectra with the help of a personal computer"

MEDICAL PROGRESS TECHNOLOGY, vol. 8, no. 2, 1981, pages 77-82, Springer-Verlag, Berlin, DE; A. MORGUET et al.: "Microcomputer-based measurement of beat-to-beat intervals and analysis of heart rate variability"

(74) Mandataire: **Mongrédien, André et al** c/o BREVATOME 25, rue de Ponthieu F-75008 Paris(FR)

## Description

La présente invention concerne un dispositif de surveillance de l'activité d'un organe du corps humain. Elle s'applique à la surveillance de l'activité d'organes tels que l'estomac, les intestins, etc. Elle permet à un patient de contrôler volontairement la motricité de l'organe étudié par une technique de contre-réaction connue sous le nom de biorétroaction. Elle permet en fait de connaître le niveau d'activité d'un organe, et elle permet au patient de contrôler ce niveau.

La biorétroaction digestive consiste pour un patient, par exemple à visualiser une courbe représentative de l'activité gastrique ; le patient peut alors contrôler cette activité par contraction ou décontraction musculaire, ou tout simplement par une plus ou moins grande relaxation psychique.

La visualisation d'une courbe représentative de cette activité, notamment l'activité gastrique, est très difficile puisque cette activité n'évolue que dans le domaine des très basses fréquences (fréquences inférieures à 0,2 hertz).

Il n'existe pas actuellement d'appareil performant permettant d'effectuer, en temps réel, une biorétroaction pour des organes dont l'activité se situe dans le domaine des très basses fréquences, notamment la biorétroaction digestive.

Les appareils actuellement connus qui permettent d'effectuer des biorétroactions, notamment digestives, ne sont pas performants et causent des traumatismes pour le patient. En effet, les appareils mesurant les rythmes digestifs par exemple utilisent de sondes gastriques ou coliques, des sondes à ballonnet, des microballons sondes, etc., qui sont introduits dans l'estomac du patient ; ceci provoque chez le patient un désagrément qui modifie et perturbe la motricité gastrique ou colique, ce qui rend la biorétroaction peu significative.

Les dispositifs connus, par exemple du "Medical and Biological Engineering; vol. 13, 1975, pages 266-271, Tonkovic et al.;- Une application de l'analyse spectrale dans le traitement des signaux electrosplanchrographiques-," qui permettent de surveiller l'activité d'un organe du corps humain mais qui ne permettent pas, notamment d'effectuer une biorétroaction digestive, comprennent généralement deux capteurs placés à proximité de l'organe à surveiller ; ces capteurs fournissent en différentiel sur une sortie, un signal électrique représentatif de l'activité de cet organe. Ce dispositif connu comprend aussi des moyens d'amplification et de filtrage, reliés par une entrée à la sortie du capteur ; ces moyens d'amplification et de filtrage fournissent sur une sortie un signal amplifié et filtré du signal d'entrée. Ce signal est fourni à un convertisseur analogique-numérique qui fournit sur une sortie des valeurs numériques représentatives du signal analogique amplifié et filtré. Ces valeurs numériques sont appliquées à des moyens de traitement fournissant sur une sortie un signal caractéristique de l'activité de l'organe. Des moyens d'exploitation visuelle, par exemple, reçoivent ce signal caractéristique pour que le patient puisse contrôler l'organe étudié par biorétroaction. Ces dispositifs connus de surveillance ne peuvent être utilisés que pour des organes dont l'activité (rythme) est traduite par des signaux de basses ou moyennes fréquences, mais non par des signaux de très basses fréquences (cas du système gastrique).

L'invention a pour but de remédier à ces inconvénients et notamment de réaliser un dispositif de surveillance de l'activité d'un organe du corps humain, permettant à un patient de contrôler l'activité de cet organe par biorétroaction, cette activité évoluant dans le domaine des très basses fréquences.

L'invention a pour objet un dispositif de surveillance de l'activité d'un organe du corps humain, comprenant au moins une paire de capteurs montés en différentiel, destinés à être placés à proximité de l'organe à surveiller pour fournir en différentiel sur leurs sorties respectives des signaux électriques représentatifs de l'activité de cet organe, des moyens d'amplification et de filtrage reliés par des entrées respectives aux sorties des capteurs, et fournissant sur une sortie un signal différentiel amplifié et filtré des signaux d'entrées, un convertisseur analogique-numérique relié par une entrée à la sortie des moyens d'amplification et de filtrage pour fournir sur une sortie des valeurs numériques correspondant au signal analogique amplifié et filtré d'entrée, des moyens de traitement reliés par une entrée à la sortie du convertisseur pour traiter les valeurs numériques et pour fournir sur au moins une sortie un signal caractéristique de l'activité de l'organe, et des moyens d'exploitation visuelle et/ou sonore de ce signal caractéristique, reliés à la sortie des moyens de traitement, caractérisé en ce que les moyens de traitement comprennent un processeur dont une entrée et une sortie constituent respectivement l'entrée et la sortie des moyens de traitement, des moyens de mémorisation reliés au processeur pour enregistrer lesdites valeurs numériques à traiter et pour enregistrer un programme de traitement de ces valeurs numériques par échantillonnage correspondant à chaque période d'une succession de périodes prédéterminées, le début d'une période étant décalé d'un intervalle de temps prédéterminé par rapport au début de la période précédente de ladite succession, le programme de traitement calculant la transformée de Fourier de ces valeurs numériques sur chaque période pour obtenir dans chaque intervalle de temps prédéterminé, le spectre de fréquences du signal d'entrée, ce programme commandant ensuite, dans la bande de fréquence spécifique de l'organe pour chaque spectre, la sélection du pic du spectre pour chaque spectre, la sélection du pic du spectre correspondant à l'organe à étudier et la recherche du maximum d'amplitude de ce pic, les moyens de traitement fournissant sur leur sortie un signal représentatif de l'évolution des maximums d'amplitudes des pics des spectres obtenus dans les intervalles de temps successifs, ce signal étant caractéristique de l'activité de l'organe.

Selon une autre caractéristique, les moyens d'exploitation visuelle sont constitués par un ensemble de visualisation et/ou une imprimante, relié à la sortie des moyens de traitement.

Selon une autre caractéristique, les moyens d'exploitation sonore comprennent un amplificateur relié à la sortie des moyens de traitement et un haut-parleur relié à une sortie de cet amplificateur pour fournir des sons dont la tonalité dépend de l'évolution du signal caractéristique.

Selon un autre mode de réalisation du dispositif de l'invention, celui-ci comprend une pluralité de capteurs placés à proximité de l'organe, les moyens d'amplification et de filtrage comportant pour chaque capteur une voie d'amplification et de filtrage dont une entrée est reliée la sortie du capteur correspondant, cette voie d'amplification et de filtrage fournissant sur une sortie un signal amplifié et filtré du signal d'entrée, et un multiplexeur analogique ayant des entrées respectivement reliées aux sorties des voies d'amplification et de filtrage et une entrée reliée à une sortie de commande de multiplexage du calculateur, une sortie de ce multiplexeur étant reliée à l'entrée du convertisseur analogique numérique.

Les caractéristiques et avantages de l'invention ressortiront mieux de la description qui va suivre donnée en référence aux dessins annexés dans lesquels :

- la figure 1 représente schématiquement un mode de réalisation du dispositif conforme à l'invention,
- la figure 2 représente schématiquement un autre mode de réalisation du dispositif conforme à l'invention,
- la figure 3A est un chronogramme du signal analogique fourni par un capteur utilisé dans le dispositif de l'invention,
- la figure 3B représente le spectre de Fourier des fréquences du signal analogique de la figure 3A, pour une période d'échantillonnage de ce signal,
- la figure 3C est un chronogramme représentant l'évolution des maximums des pics du spectre de Fourier, au cours du temps du signal analogique fourni par le capteur, lorsque ce signal subit un traitement de Fourier, par périodes successives égales et décalées d'un intervalle de temps prédéterminé.

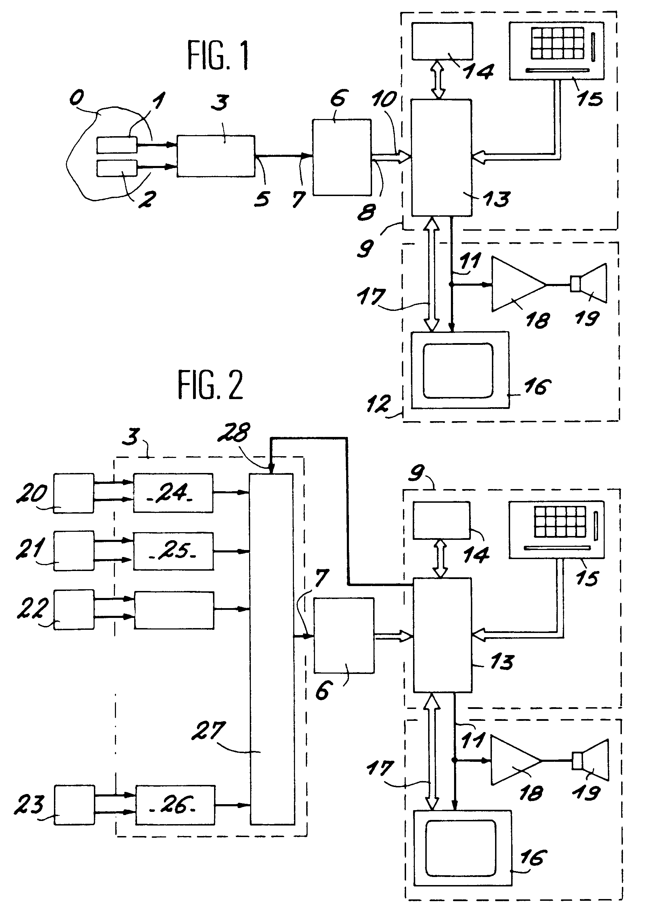

Dans le mode de réalisation représenté schématiquement sur la figure 1, le dispositif de l'invention comprend une paire de capteurs 1, 2, moulés en différentiel, placés à proximité de l'organe 0 à surveiller et fournissant, en différentiel sur leurs sorties respectives, des signaux électriques analogiques représentatifs de l'activité de cet organe. Ces capteurs ne sont pas décrits ici en détail puisqu'ils sont bien connus dans l'état de la technique et qu'ils peuvent être par exemple un capteur de type utilisé dans l'étude du rythme cardiaque.

Le dispositif comprend aussi des moyens différentiels d'amplification et de filtrage 3, reliés par leurs entrées respectives aux sorties des capteurs. Ces moyens d'amplification et de filtrage fournissent sur une sortie 5 un signal amplifié et filtré des signaux différentiels d'entrées.

Un convertisseur analogique-numérique 6 est relié par une entrée 7 à la sortie 5 des moyens d'amplification et de filtrage 3. Ce convertisseur fournit sur une sortie 8 des valeurs numériques correspondant au signal analogique amplifié et filtré.

Le dispositif comprend aussi des moyens de traitement 9 reliés par une entrée 10 à la sortie 8 du convertisseur, pour traiter les valeurs numériques reçues et pour fournir sur au moins une sortie 11, un signal caractéristique de l'activité de l'organe étudié.

Enfin, le dispositif comprend des moyens 12 d'exploitation visuelle et/ou sonore de ce signal caractéristique. Ces moyens d'exploitation sont reliés à la sortie 11 des moyens de traitement 9, pour recevoir le signal caractéristique.

Les moyens de traitement 9 comprennent un calculateur 13 dont une entrée et une sortie constituent respectivement l'entrée 10 et la sortie 11 des moyens de traitement. Ces moyens de traitement comprennent aussi des moyens de mémorisation 14, reliés au processeur 13, pour enregistrer les valeurs numériques à traiter et pour enregistrer les valeurs résultant de ce traitement, ainsi que pour enregistrer un programme de traitement de ces valeurs numériques ; ce programme sera décrit plus loin en détail.

Les moyens de mémorisation 14 peuvent être constitués par exemple par une ou plusieurs mémoires mortes programmables à lecture seule de type PROM ou EPROM, permettant d'enregistrer les programmes de traitement, et par au moins une mémoire à accès aléatoire de type RAM permettant d'enregistrer les valeurs numériques à traiter ainsi que les valeurs résultant de ces traitements.

Les moyens de traitement peuvent en outre comporter un clavier de commande 15.

Le programme de traitement enregistré dans les moyens de mémorisation 14 du processeur 13 permet tout d'abord d'échantillonner les valeurs numériques fournies par le convertisseur 6, selon des périodes d'une succession de périodes prédéterminées ; dans cette succession, le début d'une période est décalé d'un intervalle de temps prédéterminé, par rapport au début de la période précédente de cette succession, comme on le verra plus loin en détail.

Le programme de traitement permet en outre, sur chacune de ces périodes, d'effectuer un traitement de Fourier des valeurs numériques ; on obtient ainsi pour chaque intervalle de temps prédéterminé, le spectre de Fourier des fréquences du signal d'entrée, pour la période écoulée. Ce programme commande ensuite la recherche du maximum d'amplitude des pics de fréquence du spectre de Fourier pour chaque intervalle de temps. Les moyens de traitement fournissent sur leurs sorties un signal représentatif de l'évolution du maximum d'amplitude des pics des spectres correspondant aux intervalles de temps successifs, ce signal étant caractéristique, comme on le verra plus loin en détail, de l'activité de l'organe.

On ne décrira pas ici le programme d'échantillonnage du signal d'entrée, ni le programme de traitement de Fourier et de recherche des maximums d'amplitude, puisque ces différents programmes sont connus dans l'état de la technique.

Les moyens 12 d'exploitation visuelle ou sonore peuvent comprendre un ensemble de visualisation 16 et/ou une imprimante, relié à la sortie 11 des moyens de traitement 9. Sur cette figure, la liaison 17 permet par exemple un échange de signaux de commande entre les moyens de traitement 9 et l'ensemble de visualisation, ou entre cet ensemble et le clavier de commande 15, par l'intermédiaire des moyens de traitement 9.

Les moyens d'exploitation 12 peuvent aussi comprendre un amplificateur 18 relié la sortie 11 des moyens de traitement, et un haut-parleur 19 relié à une sortie de cet amplificateur pour fournir des sons dont la tonalité dépend de l'évolution du signal caractéristique de l'activité de l'organe étudié.

Sur l'écran des moyens de visualisation 16 apparaît une courbe C représentative de l'évolution de l'activité de l'organe étudié en fonction du temps. Le haut-parleur 19 fournit un son dont la tonalité varie en fonction de l'évolution de l'activité de l'organe étudié.

La figure 2 représente schématiquement un autre mode de réalisation du dispositif de l'invention. Les mêmes moyens portent les mêmes références sur cette figure et sur la figure 1. Dans ce mode de réalisation, le dispositif comprend une pluralité de paires de capteurs 20, 21, 22, 23, de type défini plus haut. Ces capteurs sont placés à proximité de l'organe. Les moyens d'amplification et de filtrage 3 comportent, pour chaque capteur, une voie d'amplification et de filtrage dont une entrée est reliée à la sortie de la paire de capteurs correspondants. Cette voie d'amplification et de filtrage fournit sur une sortie un signal amplifié et filtré des signaux différentiels d'entrées. Les différentes voies d'amplification et de filtrage sont représentées en 24, 25, 26 sur la figure. Les moyens d'amplification et de filtrage comportent aussi un multiplexeur analogique 27, relié aux sorties des voies d'amplification et de filtrage. Ce multiplexeur comprend une entrée de commande 28 reliée à une sortie de commande du calculateur 13, pour recevoir un signal de commande de multiplexage obtenu grâce à une horloge interne du calculateur, non représentée sur la figure.

Une sortie de ce multiplexeur est reliée à l'entrée 7 du convertisseur analogique-numérique 6.

Comme dans le mode de réalisation précédent, on distingue aussi sur cette figure les moyens de traitement 9 comportant le calculateur 13 et les moyens de mémorisation 14, ainsi que le clavier de commande 15. Les moyens d'exploitation visuelle ou sonore comprennent aussi, comme précédemment, un ensemble de visualisation 16 et/ou un amplificateur 18 relié à un haut-parleur 19.

Dans ce mode de réalisation le dispositif permet de sélectionner les signaux les plus significatifs fournis par un ou plusieurs des capteurs, de manière à effectuer une surveillance plus précise de l'activité de l'organe étudié.

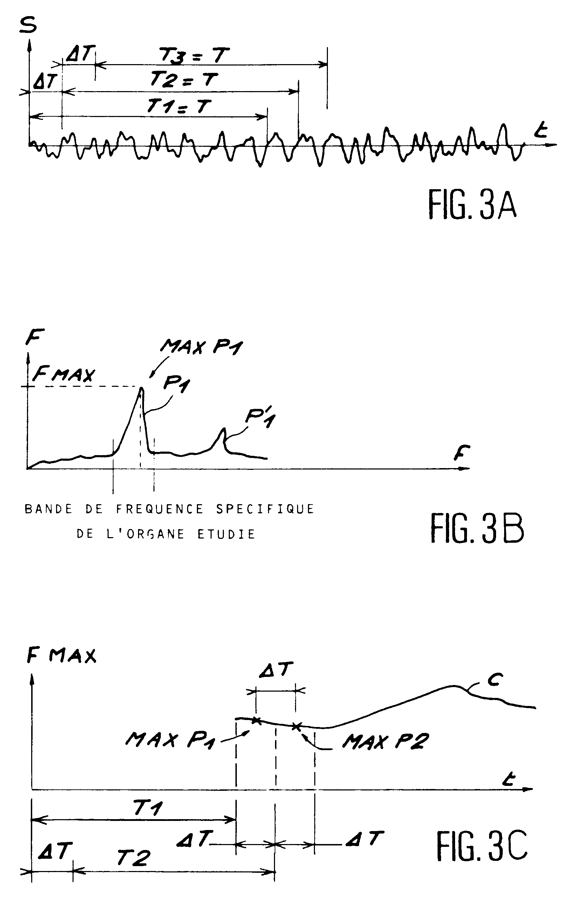

Les figures 3A, 3B, 3C vont maintenant permettre de mieux comprendre le fonctionnement du dispositif de l'invention.

Le chronogramme de la figure 3A représente les variations d'amplitude S, en fonction du temps t, du signal de sortie de l'un des capteurs du dispositif. Conformément à l'invention, ce signal est appliqué à un convertisseur analogique-numérique et les valeurs numériques obtenues sont échantillonnées sur des périodes d'une succession de périodes prédéterminées de durée T. Sur cette figure, on a représenté par T1 = T la première de ces périodes d'échantillonnage.

Selon l'invention, le début de la deuxième période d'échantillonnage T2, de même durée T, est décalé par rapport au début de la période T1 précédente, d'un intervalle de temps prédéterminé ΔT. De la même manière le début de la troisième période d'échantillonnage T3 de durée T est décalé par rapport au début de la période précédente T2, de l'intervalle de temps prédéterminé ΔT. Ainsi, les moyens de mémorisation 14 du calculateur 13 enregistrent à la sortie du convertisseur analogique-numérique 6, les valeurs numériques correspondant à ces différentes périodes décalées.

On effectue ensuite, en temps réel et pour les valeurs numériques correspondant à chacune de ces périodes, un traitement de Fourier de manière à obtenir le spectre de fréquences du signal d'entrée pour chaque période. Ce spectre de fréquences, pour l'une des périodes considérée est représenté en exemple sur la figure 3B (amplitude F du spectre en fonction des fréquences f). Ce spectre peut comporter plusieurs pics tels que P1, P'1. Dans ce spectre F on sélectionne le pic relatif à l'activité de l'organe étudié en choisissant le pic P1 qui se situe dans la bande de fréquence spécifique de l'organe étudié, tandis que le pic P'1, généralement d'amplitude inférieure, est relatif à l'activité d'un autre organe. On obtient ainsi une succession de spectres de fréquences, calculés sur des périodes T décalées les unes par rapport aux autres d'un intervalle de temps $\Delta T$. En fait, le spectre de Fourier calculé au cours de la période T1 est obtenu à la fin de cette période, au cours de l'intervalle de temps $\Delta T$ suivant. Les moyens de traitement permettent de ne retenir dans le spectre relatif à chaque période que l'amplitude maximum du pic tel que P1. Les valeurs de ces amplitudes maximum, pour les spectres correspondant à chaque période d'échantillonnage, sont bien entendu enregistrées dans les moyens de mémorisation.

La figure 3C est un chronogramme qui représente en fonction du temps t, l'évolution du maximum d'amplitude $F_{MAX}$ des pics P1 successifs, pour les différentes périodes d'échantillonnage. Cette évolution est représentée par la courbe C qui correspond au signal caractéristique fourni sur la sortie 11 du calculateur 13. Ce signal est appliqué soit aux moyens de visualisation 16, pour faire apparaître la courbe C sur l'écran de ces moyens de visualisation, soit à l'entrée de l'amplificateur 18, pour que le haut-parleur 19 fournisse un son de tonalité variable correspondant à cette courbe.

Le début de la courbe C ne peut en fait apparaître qu'à partir de la fin de la première période d'échantillonnage, puisque le calculateur 13, au cours de cette période, traite les valeurs numériques correspondant à l'échantillonnage du signal sur cette période ; le spectre de Fourier correspondant ne peut donc être obtenu qu'au cours de l'intervalle de temps $\Delta T$ débutant à la fin de cette première période T1. On a représenté en exemple sur cette figure le pic P1 d'amplitude maximum, obtenu par traitement de Fourier des valeurs numériques correspondant à l'échantillonnage du signal sur la première période T1. De la même manière, on a représenté en P2 le pic du spectre de Fourier, d'amplitude maximum, obtenu par traitement de Fourier des valeurs numériques correspondant à l'échantillonnage du signal au cours de la période T2. Ce spectre ne peut être obtenu qu'à la fin de la période T2, au cours de l'intervalle de temps $\Delta T$ qui suit la fin de cette période T2. Ainsi, la courbe C est l'enveloppe des maximums des pics des spectres de Fourier pour les périodes successives.

Le patient qui écoute le signal sonore émis par le haut-parleur 19, ou qui voit la courbe C apparaissant sur l'écran des moyens de visualisation 16 peut contrôler l'activité de l'organe étudié, par contraction ou par décontraction musculaire ou par modification de son état psychique. Le dipositif qui vient d'être décrit permet une réaction du patient en temps réel, sans que celui-ci soit incommodé par des sondes internes. Ce dispositif permet d'effectuer ainsi une biorétroaction, notamment pour le contrôle, par un patient, du niveau d'activité stomacale par exemple.

## Revendications

1. Dispositif de surveillance de l'activité d'un organe du corps humain, comprenant au moins une paire de capteurs (1, 2) montés en différentiel, destinés à être placés à proximité de l'organe (O) à surveiller pour fournir, en différentiel sur leurs sorties (2) respectives des signaux électriques représentatifs de l'activité de cet organe, des moyens (3) différentiels d'amplification et de filtrage reliés respectivement par des entrées aux sorties (3) des capteurs (1, 2) et fournissant sur une sortie (5) un signal amplifié et filtré des signaux différentiels d'entrées, un convertisseur analogique-numérique (6) relié par une entrée (7) à la sortie (5) des moyens d'amplification et de filtrage (3) pour fournir sur une sortie (8) des valeurs numériques correspondant au signal analogique amplifié et filtré d'entrée, des moyens de traitement (9) reliés par une entrée (10) à la sortie (8) du convertisseur (6) pour traiter les valeurs numériques et pour fournir sur au moins une sortie (11) un signal caractéristique de l'activité de l'organe (0), et des moyens (12) d'exploitation visuelle et/ou sonore de ce signal caractéristique, reliés à la sortie (11) des moyens de traitement (9), caractérisé en ce que les moyens de traitement (9) comprennent un processeur (13) dont une entrée (10) et une sortie (11) constituent respectivement l'entrée et la sortie des moyens de traitement (9), des moyens de mémorisation (14), reliés au calculateur (13) pour enregistrer lesdites valeurs numériques à traiter et pour enregistrer un programme de traitement de ces valeurs numériques par échantillonnage correspondant à chaque période (T) d'une succession de périodes prédéterminées, le début d'une période étant décalé d'un intervalle de temps prédéterminé ($\Delta T$) par rapport au début de la période précédente de ladite succession, le programme de traitement commandant le calcul de la transformée de Fourier de ces valeurs numériques sur chaque période (T) par le processeur, pour obtenir dans chaque intervalle de temps prédéterminé (ΔT), le spectre de fréquences du signal d'entrée, ce programme commandant ensuite, dans la bande de fréquence specifique de l'organe pour chaque spectre, la sélection du pic (P1) du spectre correspondant à l'organe à étudier et la recherche du maximum d'amplitude ($F_{max}$) du pic (P1), les moyens de traitement fournissant sur leur sortie (11) un signal (C) représentatif de l'évolution des maximums d'amplitudes ($F_{max}$) des pics des spectres obtenus dans les intervalles de temps successifs (ΔT), ce signal étant caractéristique de l'activité de l'organe (O).

2.  Dispositif selon la revendication 1, caractérisé en ce que les moyens d'exploitation visuelle sont constitués par un ensemble de visualisation (16) et/ou une imprimante, relié à la sortie (11) des moyens de traitement (9).

3.  Dispositif selon la revendication 1, caractérisé en ce que les moyens d'exploitation sonore comprennent un amplificateur (18) relié à la sortie (11) des moyens de traitement (9) et un haut-parleur (19) relié à une sortie de cet amplificateur pour fournir des sons dont la tonalité dépend de l'évolution du signal caractéristique (C).

4.  Dispositif selon la revendication 1, caractérisé en ce qu'il comprend une pluralité de capteurs (20, 21,..., 23) placés à proximité de l'organe (O), les moyens (3) d'amplification et de filtrage comportant pour chaque capteur une voie (24 ou... 26) d'amplification et de filtrage dont une entrée est reliée à la sortie du capteur correspondant, cette voie d'amplification et de filtrage fournissant sur une sortie un signal amplifié et filtré du signal d'entrée, et un multiplexeur analogique (27) ayant des entrées respectivement reliées aux sorties des voies d'amplification et de filtrage et une entrée (28) reliée à une sortie de commande de multiplexage du calculateur (13), une sortie de ce multiplexeur étant reliée à l'entrée (7) du convertisseur analogique numérique (6).

**Claims**

1.  Apparatus for monitoring the activity of an organ of the human body, comprising at least one pair of differential-connected sensors (1, 2) for positioning in the vicinity of the organ (0) to be monitored for supplying, in differential on their respective outputs (2), electric signals representative of the activity of said organ, differential amplification and filtering means (3) respectively connected by inputs to the outputs (3) of sensors (1, 2) and supplying on an output (5) an amplified and filtered signal of the differential input signals, an analog - digital converter (6) connected by an input (7) to the output (5) of the amplification and filtration means (3) for supplying on an output (8) digital values corresponding to the amplified and filtered input analog signal, processing means (9) connected by an input (10) to the output (8) of the converter (6) for processing the digital values and for supplying on at least one output (11) a signal characterizing the activity of organ (0), and visual and/or sound processing means of said characteristic signal connected to the output (11) of the processing means (9), characterized in that the processing means (9) incorporate a processor (13), whereof an input (10) and an output (11) respectively constitute the input and the output of the processing means (9), storage means (14) connected to the computer (13) for recording said digital values to be processed and for recording a programme of processing said digital values by sampling corresponding to each period (T) of a succession of predetermined periods, the start of a period being displaced by a predetermined time interval (ΔT) compared with the start of the preceding period of said succession, the processing programme controlling the calculation of the Fourier transform of said digital values on each period (T) by the processor in order to obtain in each predetermined time interval (ΔT), the frequency spectrum of the input signal, said programme then controlling in the specific frequency band of the organ. for each spectrum, the selection of the peak (P1) of the spectrum corresponding to the organ to be studied and the search for the amplitude maximum ($F_{max}$) of the peak (P1), the processing means supplying on their output (11) a signal (C) representative of the evolution of the amplitude maxima ($F_{max}$) of the peaks of the spectra obtained in successive time intervals (ΔT), said signal characterizing the activity of organ (0).

2.  Apparatus according to claim 1, characterized in that the visual processing means are constituted by a visual display means (16) and/or a printer connected to the output (11) of the processing means (9).

3.  Apparatus according to claim 1, characterized in that the sound processing means comprise an amplifier (18) connected to the output (11) of the processing means (9) and a loudspeaker (19) connected to the output of said amplifier for supplying sounds, whose tonality is dependent on the evolution of the characteristic signal (C).

4. Apparatus according to claim 1, characterized in that it comprises a plurality of sensors (20, 21, ..., 23) positioned in the vicinity of organ (0), the amplification and filtration means (3) having for each sensor an amplification and filtration channel (24, 26), whereof an input is connected to the output of the corresponding sensor, said amplification and filtration channel supplying on an output an amplified and filtered signal of the input signal, and an analog multiplexer (27) having inputs respectively connected to the outputs of the amplification and filtration channels and an input (28) connected to the multiplexing control output of computer (13), an output of said multiplexer being connected to the input (7) of the analog - digital converter (6).

**Patentansprüche**

1. Vorrichtung zur Überwachung der Aktivität eines Organs des menschlichen Körpers mit wenigstens einem Paar von Meßaufnehmern (1, 2), die differentiell montiert sind und dazu bestimmt sind, in der Nähe des zu überwachenden Organs (O) angeordnet zu werden, um an ihren jeweiligen Ausgängen (2) differentiell elektrische Signale zu erzeugen, die für die Aktivität dieses Organs repräsentativ sind; mit differentiellen Verstärker- und Filtervorrichtungen (3), die jeweils mit ihren Eingängen mit den Ausgängen (3) der Aufnehmer (1, 2) verbunden sind und auf einem Ausgang (5) ein verstärktes und gefiltertes Signal der differentiellen Eingangssignale erzeugen; mit einem Analog-Digitalwandler (6) der über einen Eingang (7) mit dem Ausgang (5) der Verstärker- und Filtervorrichtungen (3) verbunden ist, um auf einem Ausgang (8) digitale Werte zu erzeugen, die dem verstärkten und gefilterten, analogen Eingangssignal entsprechen; mit Verarbeitungsvorrichtungen (9), die über einen Eingang (10) mit dem Ausgang (8) des Wandlers (6) verbunden sind, um die digitalen Werte zu verarbeiten und um auf einem Ausgang (11) ein Signal zu erzeugen, das für die Aktivität des Organs (O) charakteristisch ist; und mit Vorrichtungen zur visuellen und/oder akustischen Auswertung dieses Signals, die mit dem Ausgang (11) der Verarbeitungsvorrichtungen (9) verbunden sind, dadurch gekennzeichnet, daß die Verarbeitungsvorrichtungen (9) einen Prozessor (13), von dem ein Eingang (10) und ein Ausgang (11) jeweils den Eingang und den Ausgang der Verarbeitungsvorrichtungen (9) bilden, und Speichervorrichtungen (14) umfassen, die mit dem Rechner (13) verbunden sind, um die zu verarbeitenden digitalen Werte und ein Bearbeitungsprogramm für diese digitalen Werte durch Abtastung entsprechend jeder Periode (T) einer Folge von vorgegebenen Perioden zu speichern, wobei der Beginn einer Periode um ein vorgegebenes Zeitintervall (ΔT) bezüglich dem Beginn der vorhergehenden Periode der Folge verschoben ist, wobei das Vearbeitungsprogramm die Berechnung der Fouriertransformierten dieser digitalen Werte für jede Periode (T) durch den Prozessor regelt, um in jedem vorgegebenen Zeitintervall (ΔT) das Frequenzspektrum des Eingangssignals zu erhalten, wobei dieses Programm anschließend in dem für das Organ spezifischen Frequenzbereich die Auswahl der Spitze (P1) des dem zu untersuchenden Organ entsprechenden Spektrums und die Suche des Amplitudenmaximums ($F_{max}$) der Spitze (P1) regelt, wobei die Verarbeitungsvorrichtungen an ihrem Ausgang (11) ein Signal (C) erzeugen, das die Entwicklung der Amplitudenmaxima (Fmax) der Spitzen der Spektren, die in den Intervallen der aufeinander folgenden Zeiten (ΔT) erhalten wurden, darstellt, wobei diese Signal für die Aktivität des Organs (O) charakteristisch ist.

2. Vorrichtung nach Anspruch 1, dadurch gekennzeichnet, daß die visuellen Auswertungsvorrichtungen aus einer Anordnung zur Sichtbarmachung (16) und/oder einem Drucker bestehen, der mit dem Ausgang (11) der Verarbeitungsvorrichtung (9) verbunden ist.

3. Vorrichtung nach Anspruch 1, dadurch gekennzeichnet, daß die akustischen Auswertungsvorrichtungen einen mit dem Ausgang (11) der Verarbeitungsvorrichtungen (9) verbunden Verstärker (18) und einen mit einem Ausgang dieses Verstärkers verbundenen Lautsprecher (19) umfassen, um Laute zu erzeugen, deren Tonhöhe von der Entwicklung des charakteristischen Signals (c) abhängt.

4. Vorrichtung nach Anspruch 1, dadurch gekennzeichnet, daß sie eine Mehrzahl von Aufnehmern (20, 21, ..., 23), die in der Nähe des Organs (O) angeordnet sind, die Verstärker- und Filtervorrichtungen (3), die für jeden Aufnehmer einen Verstärkungs- und Filterweg (24 oder ... 26) umfassen, von dem ein Eingang mit dem Ausgang des entsprechenden Aufnehmers verbunden ist, wobei dieser Verstärkungs- und Filterweg auf einem Ausgang ein verstärktes und gefiltertes Signal des Eingangssignals erzeugt, und einen Analogmultiplexer (27) umfaßt, der Eingänge, die jeweils mit den Ausgängen der Verstärkungs- und Filterwegen verbunden ist, und einen Eingang (28) besitzt, der mit einen Ausgang einer Multiplexersteuerung des Rechners (13) verbunden ist, wobei ein Ausgang dieses Multiplexers mit dem Eingang (7) des Analog-Digitalwandlers (6) verbunden ist.

FIG. 1

FIG. 2

FIG. 3A

BANDE DE FREQUENCE SPECIFIQUE
DE L'ORGANE ETUDIE

FIG. 3B

FIG. 3C